# EUROPEAN PATENT SPECIFICATION

(11) **EP 3 723 512 B1**
(45) Date of publication and mention of the grant of the patent: **19.11.2025**
(21) Application number: 18852797.2
(22) Date of filing: 11.12.2018
(51) Int. Cl.: A23L 33/105, A61K 36/9068, A61K 45/06, A61K 31/522, A61K 36/328, A61P 29/00

(54) **ASSOCIATION OF VEGETALE EXTRACTS FOR FOOD SUPPLEMENT OR MEDICAL APPLICATION**
ASSOZIATION VON PFLANZLICHEN EXTRAKTEN FÜR NAHRUNGSERGÄNZUNGSMITTEL ODER MEDIZINISCHE VORRICHTUNG
ASSOCIATION D'EXTRAITS VÉGÉTAUX POUR COMPLÉMENT ALIMENTAIRE OU DISPOSITIF MÉDICAL

(30) Priority: 13.12.2017 EP 17207018
(43) Date of publication of application: 21.10.2020
(73) Proprietor: Specchiasol S.r.l., 37012 Bussolengo (VR) (IT)
(72) Inventor: SCIALPI, Antonio, 37012 BUSSOLENGO (VR) (IT)
(74) Representative: Petraz, Gilberto Luigi
(86) International application number: PCT/IB2018/059864
(87) International publication number: WO 2019/116218

(56) References cited:
- EP-A1- 3 130 336
- WO-A1-2006/042479
- WO-A1-2017/066474
- WO-A2-2009/040824
- WO-A2-2011/106432
- CN-A- 103 610 070
- CN-A- 105 918 912
- CN-A- 106 798 906
- CN-B- 102 078 520
- US-A1- 2008 057 162
- US-A1- 2015 182 548
- DATABASE EPODOC [online] EUROPEAN PATENT OFFICE, THE HAGUE, NL; 21 October 2013 (2013-10-21), SYED TANVIR ALI DR [IN]; GURMUKH SINGH GHULDU [IN]: "A NOVEL COMPOSITION FOR ARTHRITIS", XP002790825, Database accession no. IN-3120DE2013-A
- KAUR G ET AL: "ANTIOBESITY EFFECT OF A POLYHERBAL FORMULATION, OB-200G IN FEMALE RATS FED ON CAFETARIA AND ATHEROGENIC DIETS", INDIAN JOURNAL OF PHARMACO, MEDKNOW PUBLICATIONS AND MEDIA PVT. LTD, IN, vol. 32, no. 5, 1 October 2000 (2000-10-01), pages 294 - 299, XP009060472, ISSN: 0253-7613

## Description

The present invention relates to an association of plant extracts for use as a food supplement or medical devices in general.

In particular, the subject invention relates to a innovative association of two particular plant extracts, namely myrrh (commiphora myrrha) and ginger (zingiber officinalis) together with caffeine, or of extracts containing functional principles listed above, titrated and standardized capable of moderating and/or making tolerable painful sensations and disorders as headaches, pain depending on fever, joint pains, muscle pains in general, lumbosciatalgia, menstrual pains and the like, to which the following description will make explicit reference.

The purpose of the present invention is to provide an associative composition defined by a mixture able to overcome the above mentioned disorders and painful sensations without causing gastric damage.

The structural and functional characteristics of the present invention and its advantages with respect to the known prior art will become even clearer and more evident from the following claims, and in particular from an examination of the following description.

The composition object of the present invention includes the association of the following extracts:
- dry extract and/or myrrh liquid (commiphora myrrha), capable of supplying quantities of furanodienes per daily dose ranging from 1 mg to 50 mg from dried extracts titrated from 1% to 40% in furanodienes;
- dry extract and/or ginger liquid (zingiber officinalis), capable of supplying gingerols per daily dose ranging from 1 mg to 50 mg from dried extracts titrated between 1% and 40% in gingerols; and
- caffeine.

In this way an associative mixture is created able to moderate and/or to make tolerable painful sensations and disorders such as headaches, pain dependent on fever, joint pains, muscular pains in general, lumbosciatalgia, menstrual pains and the like, eliminating the gastric damage which some non-steroidal analgesic drugs (Aspirin, Nimesulide, Ibuprofen, naproxen etc.) currently in use can determine.

The aforementioned non-steroidal analgesic drugs in fact act by inhibiting some enzymes (COX) which govern the synthesis of some molecules called prostaglandins which have the function of being mediators in the transmission of painful impulses, by interfering with the synthesis of prostaglandins.

Unfortunately, such prostaglandins in addition to mediating the pain symptoms also mediate the synthesis of an important layer of mucus which protects the stomach from damaging gastric juices and expose the gastric mucosa to important inflammations (gastritis) which then give rise to major ulcers.

In other words, such drugs are not able to act on the causes of the disturbances but only on the symptom.

Clinical studies in support of the analgesic activities of the extract being part of the association in object, to which the present invention makes explicit and direct reference, are:
- Phytother Res. 2014 Mar; 28 (3): 412-5. doi: 10.1002/ptr.4996. Epub 2013 May 9 "Comparison between the efficacy of ginger and sumatriptan in the ablative treatment of the common migraine. " Maghbooli M, Golipour F, Moghimi Esfandabadi A, Yousefi M;
- Headache 2011 Jul-Aug; 51 (7): 1078-86. doi: 10.1111 / j.1526-4610.2011.01910.x. Epub 2011 Jun 1. "A double-blind placebo-controlled pilot study of sublingual feverfew and ginger (LipiGesic ™M) in the treatment of migraine" ) Cady RK, Goldstein J, Nett R, Mitchell R, Beach ME, Browning R;
- J Ethnopharmacol. 1990 Jul; 29 (3): 267-73. "Ginger (Zingiber officinale) in migraine headache . "Mustafa T, Srivastava KC;
- Int J Physiol Pathophysiol Pharmacol. 2014 Jul 12; 6 (2): 125-36. eCollection 2014. "Active ingredients of ginger as a potential candidate in the prevention and treatment of diseases via modulation of biological activities." Rahmani AH , Shabrmi FM, Aly SM;
- BioMed Research International Volume 2017 (2017), Article ID 3804356, 11 pages https://doi.org/10.1155/2017/3804356 Clinical Study "A Pilot Study on Bioactive Constituents and Analgesic Effects of MyrLig®, a Commiphora myrrha Extract with a High Furanodiene Content." Antonio Germano, _ Andrew Occhipinti, _ Francesca Barbero, and Massimo E. Maffei ;
- Cephalalgia. 2014 Nov; 34 (13): 1070-8. doi: 10.1177 / 0333102414530527. Epub 2014 Apr 14. "Results of a multicenter, double-blind, randomized, parallel-group, placebo-controlled, single-dose study comparing the fixed combination of acetaminophen, acetylsalicylic acid, and caffeine with ibuprofen for acute treatment migraine." Goldstein J, Hagen M, Gold M ;
- J Headache Pain. 2017 Oct 24; 18 (1): 107. doi: 10.1186 / s10194-017-0806-2.

"Caffeine in the management of patients with headache."

Lipton RB, Diener HC, Robbins MS, Garas SY, K Patel.

It is also possible to refer to the following texts and graphs, incorporated directly in the present description for clarity:
- *ACTIVITY OF MIRRA EXTRACT.*

Male and female volunteers were selected based on different categories of pain:
HEADACHE
PAIN DEPENDENT FROM FEVER
ARTICOLAR PAINS
MUSCLE PAIN
LOMBOSCIATALGIA
MENSTRUAL PAINS

Volunteers were asked to compare the effects of Mirrh extract with the drug which they regularly use to treat specific pain (DI = diclofenac; KE = ketoprofen; IB = ibuprofen; PA = paracetamol; TR = tramadol; KT = ketorolac). Volunteers were asked to evaluate the effects of the extract on a scale from 0 to 10, where 0 indicates no effect and 10 indicates an effect similar to that of the drug they used to treat pain. 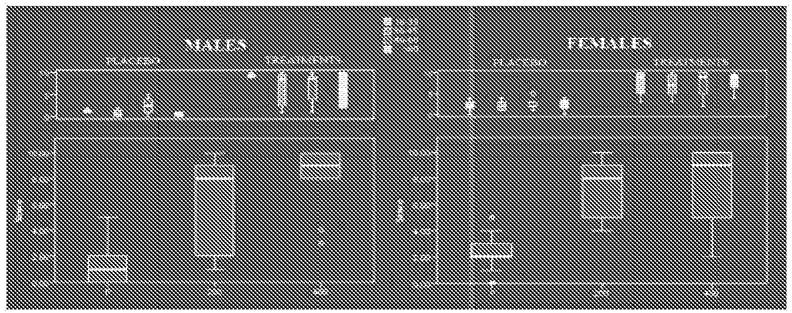
- *EXTRACT OF MIRRA (Commiphora myrra) TITRATED AND STANDARDIZED IN FURANODIENES.*

The chemical composition of the dried extracts of Mirra includes several furanodienes, such as the bioactive compounds curzerene, furanoeudesma-1,3-diene and lindestrene (Figure 1). The presence of these three main furanodienes is typical of the genus *Commiphora* and these compounds are primarily responsible for the analgesic effects of myrrh extracts.

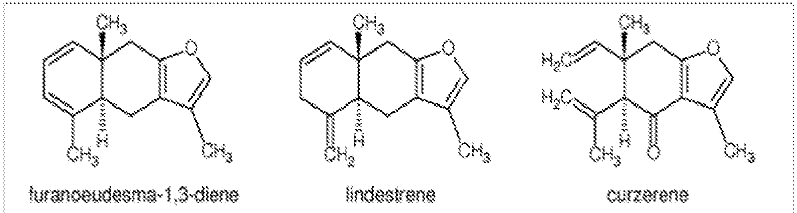

Myrrh extracts are analyzed by gas chromatography (GC) coupled to mass spectrometry (MS) for the qualitative analysis of compounds and by means of GC coupled with flame ionization detector (FID) for quantitative analysis. Figure 2 shows a typical GC-MS profile of a dry extract of Mirra :

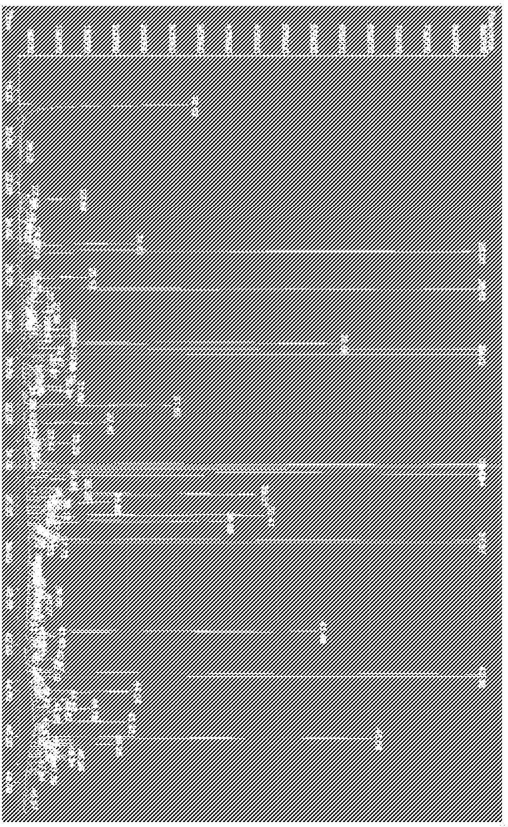

### Figure 2: GC-MS chromatographic profile of a Mirra extract

| **MOLECULE CONTENTS (g/Kg⁻¹)** | | % **FURANODIENES** |
|---|---|---|
| Curzerene | 17.93 (0.20) | 12.31 (0.05) |
| Furanoeudesma-1,3-diene | 27.44 (0.17) | 18.84 (0.02) |
| Lindestrene | 9.08 (0.06) | 6.23 (0.01) |
| Diidrolinderalattone | 1.91 (0.02) | 1.31 (0.02) |
| Acethoxy-furanodiene (MW = 274) | 0.87 (0.05) | 0.59 (0.03) |
| Acethoxy-furanodiene (MW = 232) | 1.85 (0.04) | 1.27 (0.03) |
| TOTAL | 59.07 (0.43) | 40.56 (0 |

## Claims

1. Association of plant extracts including commiphora myrrh extract mixed with ginger extract (zingiber officinalis) and caffeine.

2. Association according to claim 1, wherein said extracts of myrrh and ginger are both titrated and standardized dried extracts and dry extracts without titration.

3. Food supplement comprising the association according to one or more of the previous claims.

## Patentansprüche

1. Assoziation von Pflanzenextrakten, umfassend Commiphora Myrrha-Extrakt, der mit Ingwer-Extrakt (Zingiber Officinalis) und Koffein gemischt ist.

2. Assoziation nach Anspruch 1, worin die genannten Extrakte von Myrrhe und Ingwer beide titriert und standardisierte getrocknete Extrakte und trockene Extrakte ohne Titration sind.

3. Nahrungsergänzungsmittel umfassend die Assoziation nach einem oder mehreren von den vorhergehenden Ansprüchen.

## Revendications

1. Association d'extraits de plantes dont l'extrait de myrrhe de commiphora mélangé à de l'extrait de gingembre (zingiber officinale) et de la caféine.

2. Association selon la revendication 1, dans laquelle lesdits extraits de myrrhe et de gingembre sont à la fois des extraits séchés titrés et standardisés et des extraits secs sans titrage.

3. Complément alimentaire comprenant l'association selon l'une ou plusieurs des revendications précédentes.
